## Europäisches Patentamt
## European Patent Office
## Office européen des brevets

(19)

(11) Numéro de publication: **0 210 935**
**B1**

(12) **FASCICULE DE BREVET EUROPÉEN**

(45) Date de publication du fascicule du brevet:
**04.10.89**

(51) Int. Cl.⁴: **C 07 C 67/08,** C 07 C 69/63

(21) Numéro de dépôt: **86420196.7**

(22) Date de dépôt: **18.07.86**

(54) Procédé de préparation d'esters de l'acide trifluoroacétique ou trichloroacétique.

(30) Priorité: **26.07.85 FR 8511436**

(43) Date de publication de la demande:
**04.02.87 Bulletin 87/6**

(45) Mention de la délivrance du brevet:
**04.10.89 Bulletin 89/40**

(84) Etats contractants désignés:
**AT BE CH DE FR GB IT LI LU NL SE**

(56) Documents cité:
**FR-A-1 452 390**

(73) Titulaire: **RHONE- POULENC CHIMIE, 25, quai Paul Doumer, F-92408 Courbevoie Cédex (FR)**

(72) Inventeur: **Desbois, Michel, 526, Chemin du Bois, F-69140 Rillieux (FR)**
Inventeur: **Amiet, Louis, 10, quai St Vincent, F-69001 Lyon (FR)**

(74) Mandataire: **Le Pennec, Magali, RHONE- POULENC INTERSERVICES Service Brevets Chimie 25, Quai Paul Doumer, F-92408 Courbevoie Cédex (FR)**

## Description

La présente invention concerne un procédé de préparation d'esters de l'acide trifluoroacétique ou trichloroacétique. Elle concerne plus particulièrement un procédé de préparation d'esters de l'acide trifluoroacétique ou trichloroacétique et d'alcools non perfluorés.

Les procédés d'estérification de l'acide trifluoroacétique connus dans l'art antérieur sont très peu nombreux. Il est connu d'après la publication Research Disclosure n° 244 032 de préparer ces esters de l'acide trifluoroacétique par un procédé en 4 étapes selon lesquelles:

- on condense l'alcool de formule ROH, dans laquelle R représente un radical alkyle contenant 1 à 5 atomes de carbone, avec l'acide trifluoroacétique en présence d'un acide minéral fort (HCl, $H_2SO_4$ ou $H_3PO_4$) à une température de 25 à 110°C;
- on refroidit le mélange obtenu et on élimine la phase aqueuse;
- on traite la phase organique avec de l'acide phosphorique et/ou de l'acide sulfurique concentré puis, on poursuit la réaction entre l'acide et l'alcool à 25 - 110°C;
- on isole l'ester par distillation.

Ce procédé présente d'une part l'inconvénient de nécessiter un nombre d'étapes important, d'autre part la récupération de l'acide sulfurique anhydre est difficile. L'acide sulfurique est en outre souvent un milieu trop oxydant, ce qui entraîne des diminutions de rendements réactionnels.

La présente invention a su vaincre les inconvénients de l'art antérieur et a pour objet un procédé de préparation d'esters de l'acide trifluoroacétique ou trichloroacétique caractérisé en ce que l'on met en présence l'acide trifluoroacétique ou trichloroacétique et un alcool aliphatique non perfluoré au sein de l'acide fluorhydrique liquide et en ce que l'on sépare l'ester obtenu par décantation.

L'un des principaux avantages du procédé selon l'invention est le fait que, d'une part, aucune distillation n'est nécessaire en fin de réaction, en effet, une simple décantation suffit pour séparer l'ester obtenu; d'autre part, l'acide fluorhydrique présente une viscosité bien inférieure à celle de l'acide sulfurique, ce qui facilite la mise en oeuvre technique.

Les alcools utilisables pour l'estérification sont tous les alcools aliphatiques linéaires ou ramifiés non perfluorés, de formule générale:

R - $C_nH_{2n}$ OH dans laquelle n est compris entre 1 et 12 et, de préférence entre 1 et 5.

R est choisi parmi l'hydrogène ou un substituant aromatique.

On peut citer, mais non limitativement, parmi les alcools utilisables, le méthanol, l'éthanol, l'isopropanol, le tertiobutanol, le pentanol 2, l'alcool benzylique.

L'acide fluorhydrique utilisé pour l'estérification est anhydre ou aqueux. Lorsque l'acide fluorhydrique utilisé est aqueux, on préfère utiliser de l'acide fluorhydrique contenant au plus 60 % d'eau (acide fluorhydrique à 40 %).

Pour la décantation, on ajoute éventuellement de l'eau au mélange réactionnel.

Pour une meilleure réalisation de l'invention, on préfère utiliser une quantité d'alcool et d'acide trifluoroacétique ou trichloroacétique environ stoechiométrique.

De façon tout à fait avantageuse on utilise notamment une quantité d'acide fluorhydrique tel que le rapport molaire de l'alcool ou de l'acide trifluoroacétique ou trichloroacétique par rapport à l'acide fluorhydrique soit compris de préférence entre 0,1 et 10 et encore plus particulièrement entre 0,3 et 5.

La température de réaction est avantageusement comprise entre 0 et 50°C et encore plus avantageusement entre 10 et 20°C.

La pression à laquelle l'estérification est réalisée est de préférence la pression atmosphérique.

On peut citer parmi les produits obtenus par le procédé de la présente invention, le trifluoroacétate de méthyle, d'éthyle, d'isopropyle, de tertiobutyle.

Ces produits sont utilisés comme intermédiaires de synthèse dans l'industrie pharmaceutique ou phytosanitaire. (US-4 358 604).

L'invention va être maintenant plus complètement décrite à l'aide des exemples suivants qui ne doivent être considérés en aucun cas comme limitatifs de l'invention.

## Exemple 1

Dans un réacteur en polyéthylène de 250 ml, on introduit successivement 57 g (0,5 m) d'acide trifluoroacétique, 23 g (0,5 m) d'éthanol absolu et 19 g d'acide fluorhydrique à 50 %. Après 3 heures d'agitation à température ambiante (environ 15°C), le mélange réactionnel se divise en deux phases non miscibles que l'on sépare par décantation.

On récupère ainsi 66 g de trifluoroacétate d'éthyle, soit un rendement de 93 %.

## Exemple 2

On opère de manière identique à l'exemple 1 avec les conditions et produits suivants:

- Acide trifluoroacétique          57 g (0,5 m),
- Pentanol-2                        44 g (0,5 m),
- Acide fluorhydrique anhydre       20 g (1 m),
- Température                       10 - 15°C,
- Durée                            3 heures.

Après décantation, on récupère 81 g de trifluoroacétate de pentyle-2, soit un rendement de 88 %.

## Exemple 3

On opère de manière identique à l'exemple 1 mais en rajoutant un peu d'eau après la réaction, ce qui provoque alors la démixion, et avec les conditions et produits suivants:

- Acide trifluoroacétique       57 g (0,5 m),
- Ethanol                       23 g (0,5 m),
- Acide fluorhydrique anhydre   20 g (1 m),
- Eau                           20 g (1,1 m),
- Température                   10°C,
- Durée                         3 heures 30 mn.

Après décantation, on récupère 66 g de trifluoroacétate d'éthyle, soit un rendement de 93 %.

## Exemple 4

On opère de manière identique à l'exemple 3, avec les conditions et produits suivants:

- Acide trifluoroacétique       57 g (0,5 m),
- Méthanol                      16 g (0,5 m),
- Acide fluorhydrique anhydre   10 g (0,5 m),
- Eau                           16 g (0,9 m),
- Température                   10 - 15°C,
- Durée                         3 heures.

Après décantation, on récupère 52 g de trifluoroacétate de méthyle, soit un rendement de 80 %.

## Exemple 5

On opère de manière identique à l'exemple 3 avec les conditions et produits suivants:

- Acide trifluoroacétique       57 g (0,5 m),
- Isopropanol                   30 g (0,5 m),
- Acide fluorhydrique anhydre   30 g (1,5 m),
- Eau                           30 g (1,7 m),
- Température                   15°C,
- Durée                         3 heures.

Après décantation, on récupère 74 g de trifluoroacétate d'isopropyle, soit un rendement de 95 %.

## Exemple 6

On opère de manière identique à l'exemple 3 avec les conditions et produits suivants:

- Acide trifluoroacétique       57 g (0,5 m),
- Ethanol                       23 g (0,5 m),
- Acide fluorhydrique anhydre   5 g (0,25 m),
- Eau                           20 g (1,1 m),
- Température                   10°C,
- Durée                         2 heures 30 mn.

Après décantation, on récupère 57 g de trifluoroacétate d'éthyle, soit un rendement de 80 %.

## Exemple 7

On opère de manière identique à l'exemple 3 avec les conditions et produits suivants :

- Acide trifluoroacétique       57 g (0,5 m),
- Ethanol                       23 g (0,5 m),
- Acide fluorhydrique           66 % 15 g,
- Eau                           10 g (0,55 m),
- Température                   20°C,
- Durée                         3 heures.

Après décantation, on récupère 64,7 g de trifluoroacétate d'éthyle, soit un rendement de 91 %.

## Exemple 8

On opère de manière identique à l'exemple 3 avec les conditions et produits suivants:

- Acide trichloroacétique       81,5 g (0,5 m),
- Ethanol                       23 g (0,5 m),
- Acide fluorhydrique anhydre   5 g (0,25 m),
- Eau                           30 g (1,67 m),
- Température                   20°C
- Durée                         3 heures.

Après décantation, on récupère 90 g de trichloroacétate d'éthyle soit un rendement de 94 %.

## Exemple 9

On opère de manière identique à l'exemple 1 avec les conditions et produits suivants:

- Acide trifluoroacétique       57 g (0,5 mole)
- Alcool laurique               93 g (0,5 mole)
- Acide fluorhydrique anhydre   5 g (0,25 mole)
- Température                   25°C
- Durée                         4H 30

Après décantation, on récupère 118 g de trifluoroacétate de lauryle soit un rendement de 83,7 %.

## Exemple 10

On opère de manière identique à l'exemple 1 avec les conditions et produits suivants:

| | | |
|---|---|---|
| - Acide trifluoroacétique | 57 g | (0,5 mole) |
| - Ethanol | 23 g | (0.5 mole) |
| - Acide fluorhydrique à 40 % | 20 g | |
| - Température | 20°C | |
| - Durée | 5 H 30 | |

Après décantation, on récupère 55 g de trifluoroacétate d'éthyle, soit un rendement de 77,5 %.

## Revendications

1. Procédé de préparation d'esters de l'acide trifluoroacétique ou trichloroacétique, caractérisé en ce que l'on met en présence l'acide trifluoroacétique ou trichloroacétique et un alcool aliphatique non perfluoré au sein de l'acide fluorhydrique liquide et en ce que l'on sépare l'ester obtenu par décantation.

2. Procédé selon la revendication 1 caractérisé en ce que l'alcool aliphatique non perfluoré a pour formule générale $R - C_n H_{2n} OH$ dans laquelle n est compris entre 1 et 12, et de préférence entre 1 et 5, et R est choisi parmi l'hydrogène ou un substituant aromatique.

3. Procédé selon les revendications 1 et 2 caractérisé en ce que l'acide fluorhydrique contient au maximum 60 % d'eau.

4. Procédé selon la revendication 1 caractérisé en ce que le rapport molaire de l'alcool à l'acide trifluoroacétique ou trichloroacétique est d'environ 1.

5. Procédé selon les revendications 1 et 3 caractérisé en ce que le rapport molaire de l'alcool ou de l'acide trifluoroacétique ou trichloroacétique à l'acide fluorhydrique est compris entre 0,1 et 10, et de préférence entre 0,3 et 5.

6. Procédé selon la revendication 1 caractérisé en ce que la température de réaction est comprise entre 0 et 50°C et de préférence, entre 10 et 20°C.

## Patentansprüche

1. Verfahren zur Herstellung von Trifluoressigsäure- und Trichloressigsäureestern, dadurch gekennzeichnet, daß man Trifluoressigsäure oder Trichloressigsäure und einen nicht perfluorierten aliphatischen Alkohol in flüssiger Fluorwasserstoffsäure zusammengibt und den erhaltenen Ester durch Dekantieren abtrennt.

2. Verfahren nach Anspruch 1, dadurch gekennzeichnet, daß der nicht perfluorierte aliphatische Alkohol die allgemeine Formel $R - C_n H_{2n} OH$ aufweist, in der n 1 bis 12 und vorzugsweise 1 bis 5 ist und R aus Wasserstoff und einem aromatischen Substituenten ausgewählt ist.

3. Verfahren nach Anspruch 1 und 2, dadurch gekennzeichnet, daß die Fluorwasserstoffsäure im Maximum 60 % Wasser enthält.

4. Verfahren nach Anspruch 1, dadurch gekennzeichnet, daß das Molverhältnis von Alkohol zur Trifluoressigsäure oder Trichloressigsäure ungefähr 1 beträgt.

5. Verfahren nach den Ansprüchen 1 und 3, dadurch gekennzeichnet, daß das molare Verhältnis des Alkohols oder der Trifluoressigsäure oder der Trichloressigsäure zur Fluorwasserstoffsäure zwischen 0,1 uns 10 und vorzugsweise zwischen 0,3 und 5 liegt.

6. Verfahren nach Anspruch 1, dadurch gekennzeichnet, daß die Reaktionstemperatur zwischen 0 und 50°C und vorzugsweise zwischen 10 und 20°C liegt.

## Claims

1. Process for preparing trifluoroacetic or trichloroacetic acid esters, characterized in that trifluoroacetic or trichloroacetic acid is brought together with a non-perfluorinated aliphatic alcohol in liquid hydrofluoric acid and in that the ester obtained is separated by decantation.

2. Process according to claim 1, characterized in that the non-perfluorinated aliphatic alcohol has the general formula $R - C_n H_{2n} OH$, in which n is between 1 and 12, and preferably between 1 and 5, and R is chosen from hydrogen or an aromatic substituent.

3. Process according to claims 1 and 2, characterized in that the hydrofluoric acid contains at most 60 % of water.

4. Process according to claim 1, characterized in that the mole ratio of the alcohol to the trifluoroacetic or trichloroacetic acid is approximately 1.

5. Process according to claims 1 and 3, characterized in that the mole ratio of the alcohol or the trifluoroacetic or trichloroacetic acid to the hydrofluoric acid is between 0.1 and 10, and preferably between 0.3 and 5.

6. Process according to claim 1, characterized in that the reaction temperature is between 0 and 50°C, and preferably between 10 and 20°C.